# EUROPEAN PATENT APPLICATION

(11) **EP 4 717 303 A1**
(43) Date of publication of application: **01.04.2026**
(21) Application number: 24900613.1
(22) Date of filing: 03.12.2024
(51) Int. Cl.: A61N 5/06

(54) **LIGHT IRRADIATION DEVICE**

(30) Priority: 06.12.2023 JP 2023206239
(71) Applicant: Japan PBM Healing Kabushiki Kaisha, Tokyo 108-0023 (JP)
(72) Inventor: KWONG HING Alan, 75004 Paris (FR)
(74) Representative: Gislon, Gabriele
(86) International application number: PCT/JP2024/042726
(87) International publication number: WO 2025/121319

(57) **Abstract**

An object of the present invention is to provide a light irradiation device that is capable of accurately irradiating light according to affected parts and their symptoms.

A light irradiation device 1 irradiating light to an affected part, includes a main body 2 including a substrate 5, and a light-emitting part 7 that is disposed on at least one surface 5a of the substrate 5 and includes a plurality of light emitters 6 with different emission wavelengths, and a controller 3 equipped with a control part 8 that controls light emission from the light-emitting part 7. The light-emitting part 7 at least includes the first light emitters 6a emitting light with a wavelength in the first band and the second light emitters 6b emitting light with a wavelength in the second band, different from the first band. The control part 8 executes an operation by switching at least between the first mode in which the first light emitters 6a are made to emit, the second mode in which the second light emitters 6b are made to emit, and the third mode in which the first light emitters 6a and the second light emitters 6b are made to emit.

## Description

### FIELD OF THE INVENTION

The present technique relates to a light irradiation device, particularly to a light irradiation device irradiating light to a target area from the surface of skin or mucous membrane.

### BACKGROUND OF THE INVENTION

Conventionally, phototherapy has been used by irradiating light to affected parts of a patient from above the surface of the skin or mucous membrane to either promote wound healing in injuries or surgical wounds or to alleviate pain. The phototherapy involves irradiating the affected parts of the patient with light of various wavelengths in various light regions, such as the visible light region, ultraviolet light region, radiofrequency region, or infrared light region, etc., depending on the treatment method, using phototherapy equipment.

Examples of devices used in such phototherapy include a device that irradiates a laser light to a treatment target area (Patent Document 1) and a device that irradiates a monochromatic light to a treatment target area (Patent Document 2).

### PRIOR ART

### PATENT DOCUMENTS

Patent Document 1: JP H09-038221 A
Patent Document 2: JP 2001-212250 A

### SUMMARY OF THE INVENTION

### PROBLEMS TO BE SOLVED BY THE INVENTION

There are various affected parts and their symptoms that require phototherapy. Additionally, symptoms change over time. Therefore, phototherapy equipment used for phototherapy is required to deliver light irradiation according to the position of the affected part, its symptoms, and the progress of time.

Thus, an object of the present technique is to provide a light irradiation device that is capable of accurately irradiating light according to affected parts and their symptoms.

### MEANS FOR SOLVING THE PROBLEMS

In view of the above disadvantages, a light irradiation device according to the present technique includes:
a main body having a substrate, and a light-emitting part that is disposed on at least one surface of the substrate and includes a plurality of light emitters with different emission wavelengths, and
a controller equipped with a control part that controls light emission from the light-emitting part,
wherein the light-emitting part at least includes the first light emitters emitting light with a wavelength in the first band and the second light emitters emitting light with a wavelength in the second band, different from the first band, and
wherein the control part executes an operation by switching at least between the first mode in which the first light emitters are made to emit, the second mode in which the second light emitters are made to emit, and the third mode in which the first light emitters and the second light emitters are made to emit.

### ADVANTAGES OF INVENTION

The light irradiation device to which the present technique is applied is capable of differentiating emission wavelengths and cumulative light quantities between the first to third modes. Therefore, it is capable of suitably irradiating light according to the affected parts and the progression of their symptoms by switching modes.

### BRIEF DESCRIPTION OF THE DRAWINGS

Fig. 1 is a drawing showing a configuration of a light irradiation device to which the present technique is applied.
Fig. 2 is a function block diagram showing a configuration example of a main body and a controller.
Fig. 3 is a top plan view showing the light irradiation device according to the first embodiment.
Fig. 4 is a drawing showing a using state of the light irradiation device according to the first embodiment.
Fig. 5 is a drawing showing a holder of the light irradiation device according to the first embodiment, (A) is a top plan view, and (B) is a top plan view showing a state in the process of attaching a pocket.
Fig. 6 is an exploded perspective view showing the main body of the light irradiation device according to the first embodiment.
Fig. 7 is a top plan view showing the light irradiation device according to the second embodiment.
Fig. 8 is a drawing showing a using state of the light irradiation device according to the second embodiment.

### EMBODIMENTS OF THE INVENTION

A light irradiation device to which the present technique is applied is explained in detail by referring to the drawings. Incidentally, the present technique is not restricted to the following embodiments, and various modifications may be made within the scope of the gist of the present technique. The drawings are schematic, and the proportions of the dimensions may differ from those of the actual product. Specific dimensions should be determined by taking into consideration the following explanation. Furthermore, there are, of course, differences in the dimensional relations and ratios between the drawings.

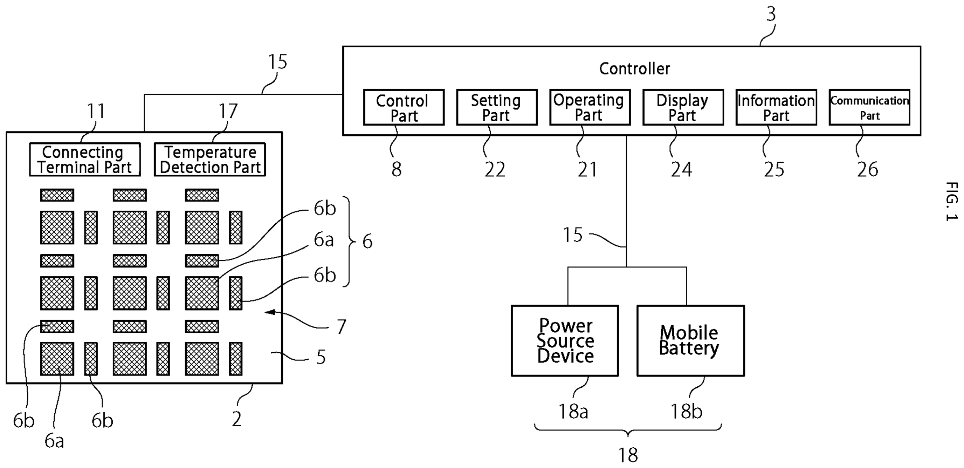

Fig. 1 is a drawing showing a configuration of a light irradiation device 1 to which the present technique is applied. As shown in Fig. 1, the light irradiation device 1 to which the present technique is applied includes a main body 2 having a substrate 5, and a light-emitting part 7 that is disposed on at least one surface of the substrate 5 and has a plurality of light emitters 6 with different emission wavelengths, and a controller 3 equipped with a control part 8 that controls light emission from the light-emitting part 7.

The light-emitting part 7 at least includes the first light emitters 6a emitting light with a wavelength in the first band and the second light emitters 6b emitting light with a wavelength in the second band. In the light-emitting part 7, the wavelength (wavelength: nm) and radiant flux (Radiant Flux: W) of the irradiation light emitted from the first light emitters 6a are different from those of the irradiation light emitted from the second light emitters 6b.

The control part 8 executes an operation by switching at least between the first mode in which the first light emitters 6a are made to emit, the second mode in which the second light emitters 6b are made to emit, and the third mode in which the first light emitters 6a and the second light emitters 6b are made to emit.

Thus, the light irradiation device 1 is capable of differentiating radiation illuminance (Irradiance: W/m²) and absorbed energy (J) for an affected part between the first to third modes. Therefore, it is capable of suitably irradiating light according to the affected parts and progression of their symptoms by switching the modes.

Each component of the light irradiation device 1 is described in detail below.

### [Main body]

The main body 2 has the substrate 5, and the light-emitting part 7 that is disposed on at least one surface of the substrate 5 and has the plurality of light emitters 6 with different emission wavelengths.

### [Substrate]

The substrate 5 is an insulating substrate, and is formed with an insulating film with flexibility, such as polyimide, or a rigid substrate, such as a glass-reinforced epoxy substrate, according to the use, for example. As the substrate 5, a flexible substrate having an excellent adhesion property to the affected part is preferable. By using the flexible substrate, even when the affected part has a curved surface, such as an arm, leg, face, or hip, the main body 2 can adhere to the affected part and irradiate light efficiently.

As the substrate 5 having flexibility, various materials can be used other than the polyimide resin film, for example, a film of fluororesin, silicone resin, PET (polyethylene terephthalate) resin, liquid crystal polymer, etc., highly reflective resin films that are obtained by coating a resin containing a white pigment (white resin, white resist, etc.) on each surface of these films, or highly reflective resin films in which a white pigment is mixed.

The area of the substrate 5 is not particularly limited, but is appropriately designed according to the application and the number and arrangement pattern of the first and second light emitters 6a and 6b. For example, a light irradiation device 40 used for the treatment of temporomandibular joint arthrosis, described below, is sized to cover the temporomandibular joint and its surrounding area (e.g., 60 to 85 mm × 115 to 120 mm). Furthermore, another light irradiation device 50 used for mucous membrane treatment in an oral cavity, described below, is sized to fit comfortably on a tongue or inside a cheek (e.g., 50 to 60 mm × 60 to 90 mm).

The thickness of the substrate 5 is not particularly limited, but is appropriately designed according to the application, for example, about 0.5 mm thick.

Incidentally, in the light irradiation device 1, one surface of the substrate 5 on which the light emitters 6 are mounted is referred to as a top surface 5a, and another surface of the substrate 5 opposite to the top surface 5a is referred to as a bottom surface 5b.

### [Light-emitting part]

The substrate 5 is formed with wiring not shown, and the light emitters 6 that configure the light-emitting part 7 are mounted on the wiring. The wiring is formed, for example, with copper-plated wiring of which surface is covered with silver plating. The wiring material is preferably low in resistance from the viewpoint of light emission efficiency. In addition, from the viewpoint of returning the light reflected from the affected part to the affected part during light irradiation and reducing the loss, a material with high surface reflectivity, for example, a material with total light-flux reflectivity of 80% or more, is preferable.

The light emitters 6 are connected to a connecting terminal part 11 provided on the top surface 5a or the bottom surface 5b of the substrate 5 via the wiring on the substrate 5. The connecting terminal part 11 is connected to the controller 3 via an electric cable 15. The light emitters 6 are controlled by the controller 3 connected to the end portion of the electric cable 15 to regulate light irradiation, e.g., irradiation ON and OFF, irradiation mode (the first light emitters 6a and/or the second light emitters 6b, time (sec), radiation illuminance (W/m²), absorbed energy (J), etc.).

### [Light emitters]

The light emitters 6 are not particularly restricted. As the light emitters 6, there are examples, such as light-emitting diode (LED), organic light-emitting diode (OLED), semiconductor laser diode (LD), polymer light-emitting diode (PLED), light-emitting polymer (LEP), optical fiber bundle, etc., or combinations thereof, but are not limited to these. The light emitters 6 are connected to the wiring, e.g., by mounting on a surface of a pad (not shown) provided on the top surface 5a of the substrate 5.

The light emitters 6 that configure the light-emitting part 7 include the first light emitters 6a emitting light with a wavelength in the first band and the second light emitters 6b emitting light with a wavelength in the second band. The wavelength in the first band is different from that in the second band. For example, the first band is within a range of 640 to 770 nm (660 ± 10%), and the second band is within a range of 770 to 1,000 nm (880 ± 10%). Incidentally, in the present specification, when there is no distinction between the first light emitters 6a and the second light emitters 6b, or when referring to both the first and second light emitters 6a, 6b, they may be referred to as "light emitters 6." The same applies when light emitters other than the first and second light emitters 6a, 6b are installed, such as the third light emitters described below.

In the light-emitting part 7, a unit is configured by combining the first light emitters 6a and the second light emitters 6b, and multiple units are provided on the top surface 5a of the substrate 5. Each configuration of the units is not particularly restricted and can be designed arbitrarily. In the example shown in Fig. 1, the units configured with one of the first light emitters 6a and two of the second light emitters 6b are arranged uniformly.

The light irradiation device 1 has at least three modes for irradiating the light emitters 6. That is, the light irradiation device 1 has the first mode in which only the first light emitters 6a are made to emit, the second mode in which only the second light emitters 6b are made to emit, and the third mode in which the first light emitters 6a and the second light emitters 6b are made to emit. The mode switching is controlled by the control part 8 provided in the controller 3 described below.

The wavelength (wavelength: nm) and radiant flux (Radiant Flux: W) of the irradiation light emitted from the first light emitters 6a are different from those of the irradiation light emitted from the second light emitters 6b. Thus, the light irradiation device 1 is capable of differentiating radiation illuminance (Irradiance: W/m²) and absorbed energy (J) for the affected part between the first to third modes by switching the modes using the control part 8, and is capable of suitably irradiating light according to the affected parts and progression of their symptoms.

In addition to these, in the light irradiation device 1, an irradiation pattern of each of the modes may be properly set. The first light emitters 6a and/or the second light emitters 6b may be uniformly lit at the same radiation illuminance, or flashed. Moreover, the irradiation position, the radiation illuminance, and the irradiation pattern may be set to vary with time.

In the light irradiation device 1, a light output (radiant flux) of the wavelength of the first band of the first light emitters 6a may be differentiated between the first mode and the third mode. For example, in the first mode, in which only the first light emitters 6a emit, the output may be made to be high, and in the third mode, in which both the first light emitters 6a and the second light emitters 6b emit, the output may be made to be low.

As the first light emitter 6a, an LED emitting red light (emission wavelength: 640 to 770 nm) is exemplified. As the second light emitter 6b, an LED emitting infrared light (emission wavelength: approximately 770 nm to 1,000 nm) is exemplified.

The light-emitting part 7 can include the third light emitters emitting light with a wavelength in the third band different from the first and second bands. As the wavelength of the third band of the third light emitters, for example, the blue band light of a wavelength (emission wavelength: 430 to 490 nm) can be used. The light irradiation device 1 can irradiate light optimally according to the affected part and its symptoms by setting the mode in which the third light emitters are used alone or with either or both the first and second light emitters 6a and 6b.

The red light (emission wavelength: 640 to 770 nm) is said to be relatively effective for alleviating pain outside the affected part. The infrared light (emission wavelength: approximately 770 nm to 1,000 nm) is said to be effective in promoting blood flow, treating bone, and alleviating pain inside the affected part. The blue light (emission wavelength: 430 to 490 nm) is said to have sterilizing effects on bacteria and disinfectant properties. When the light irradiation device 1 is used for alleviation of severe pain, and so on, the first mode to irradiate the red light only from the first light emitters 6a is selected. Since the output of the first light emitters 6a in the first mode is larger than that in the other modes, it is possible to irradiate light more suitably for pain alleviation.

### [Temperature detection part]

Incidentally, the substrate 5 may include a temperature detection part 17, such as a thermistor, for detecting the temperature of the light emitters 6. This allows the light emitters 6 to be controlled to stop the light irradiation when they are heated to a predetermined temperature or higher, thereby protecting the wearer and the main body 2.

### [Controller]

Next, the controller 3 is described, which includes the control part 8 for controlling light emission from the light-emitting part 7. As shown in Fig. 1, the controller 3 is connected to the main body 2 via the electric cable 15. Moreover, the controller 3 is connected to a power supply unit 18 via the other electric cable 15. The controller 3 is detachably connected to the main body 2 and the power supply unit 18 via the respective electric cables 15.

The power supply unit 18 can be a power source device (adapter) 18a connected to a household outlet or a mobile battery 18b containing a rechargeable or disposable battery. In this way, by separating the main body 2 from the controller 3 and the power supply unit 18, and by connecting them with the cables, the weight required when fitting the main body 2 is only the weight of the main body 2 itself, and misalignment of a fitting position can be prevented, the wearer's burden of holding the main body 2 on the affected part can be reduced, and further improving operability. Furthermore, since the main body 2 is detachable from the power cable 15, maintenance such as replacing or cleaning only the main body 2 after use is also easy.

Incidentally, the secondary battery may be built into the controller 3 or the main body 2. The secondary battery built into the controller 3 or the main body 2 can be charged when the controller 3 or the main body 2 is connected to the power source device connected to the household outlet, via the power source cable.

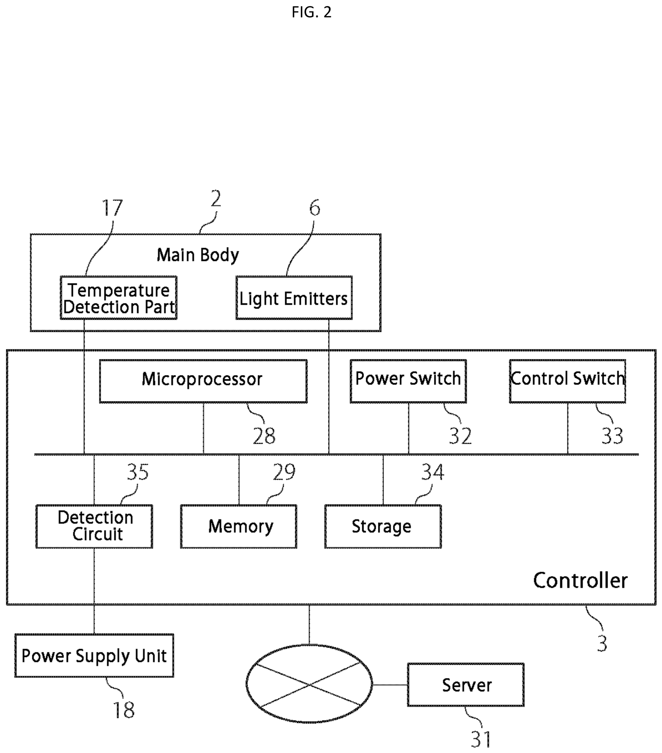

Figs. 1, 2 are respectively a function block diagram showing a configuration example of the main body 2 and the controller 3. As shown in Figs. 1, 2, the controller 3 has the control part 8 and an operating part 21, wherein the control part 8 controls the irradiation area, irradiation time, wavelength of irradiation light, radiation illuminance, etc. of the light emitters 6 based on a program, and stores a mode in which the irradiation area, irradiation time, wavelength of irradiation light, radiation illuminance, absorbed energy, etc. are set in advance, and the operation history of the intraoral light irradiation device 1 in memory and storage, and wherein the operating part 21 is equipped with a control button for operation such as operating a power source ON/OFF, turning irradiation ON/OFF, and selecting modes.

The controller 3 may be provided with a setting part 22 for manually setting the irradiation area, irradiation time, wavelength of irradiation light, radiation illuminance, absorbed energy, etc., a display part 24, such as an LCD panel, that displays the operation condition, operation history, schedule management, remaining light irradiation time, battery level, etc. of the light irradiation device 1, an information part 25 that notifies of the completion of a predetermined light irradiation, error states, and so forth, via alarms, light emission, vibration, etc., and a communication part 26 that connects to an external server 31 via the internet and performs renewal of a control program and a schedule stored in the control part 8 and a transmission of the operation history saved in the control part 8.

The control part 8 includes a microprocessor 28. The microprocessor 28 communicates with a memory 29 or a storage 34 about a program such as the first to third modes, the operation history of the light irradiation device 1, and so forth. The microprocessor 28 also saves the setting information of the setting part 22 in the memory 29 or the storage 34, and controls the irradiation according to the setting saved in the memory 29 or the storage 34.

The microprocessor 28 operates according to the operations of a power switch 32, which turns the power source ON/OFF, and a control switch 33, which operates the start/stop of irradiation. The microprocessor 28 also stops the light irradiation in response to a signal from the temperature detection part 17, such as a thermistor. Thus, when the light emitters 6 are heated to a predetermined temperature or more, it is possible to control the light irradiation device so that the light irradiation is stopped, so as to protect the wearer and the main body 2.

Furthermore, the microprocessor 28 receives an output of a detection circuit 35 for detecting an output voltage when the power supply unit 18 is configured with a mobile battery, judges a charge amount, and controls the display part 24 and the information part 25 to inform a user.

### [First embodiment]

A specific application example of the light irradiation device 1 is described below. Incidentally, the following embodiment is an application example of the light irradiation device 1, and the present invention is not restricted to this embodiment.

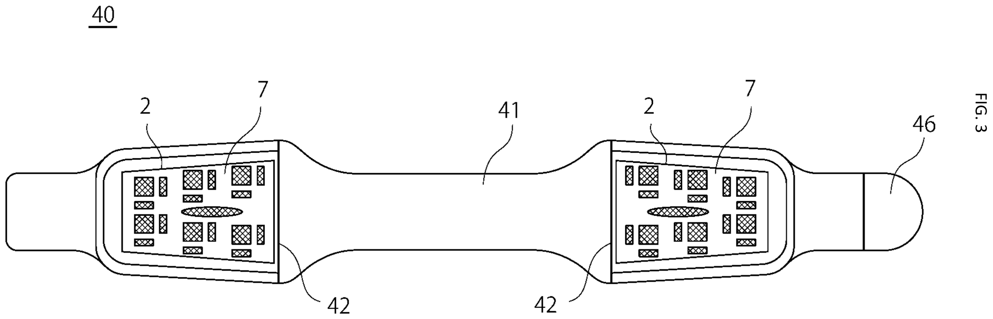

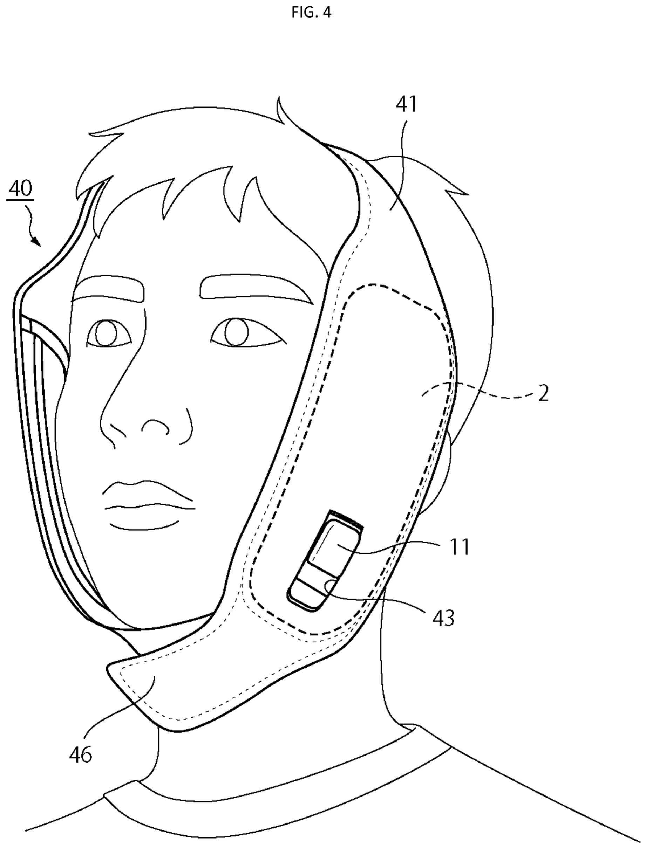

The light irradiation device 40 according to the first embodiment is used for treating temporomandibular joint arthrosis and disorders (TMJ/TMD). Fig. 3 is a top plan view showing the light irradiation device 40, and Fig. 4 is a drawing showing a using state of the light irradiation device 40. The light irradiation device 40 has the main bodies 2 and a holder 41 that stores the main bodies 2 and is wound around the face of the user to place the main bodies 2 at each predetermined position on the face.

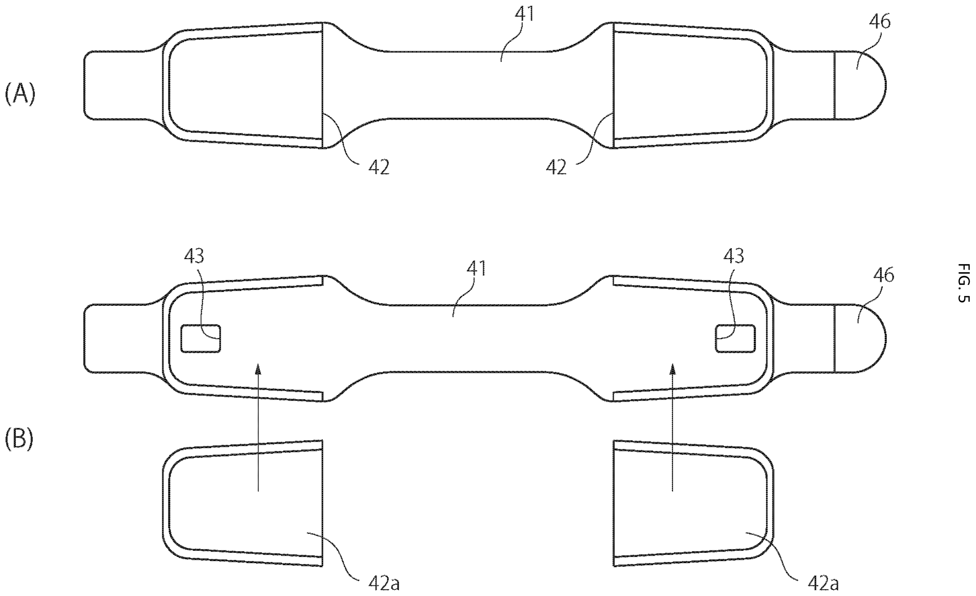

Fig. 5 is a drawing showing the holder 41, (A) is a top plan view, and (B) is a top plan view showing a state in the process of attaching a pocket. The holder 41 is formed in a beltlike shape, and is formed with two pockets 42, each of which houses the main body 2, to be separately provided in a longitudinal direction. The light irradiation device 40 stores the main bodies 2 in the pockets 42 respectively so that each of the light-emitting parts 7 faces the front side of the corresponding pocket 42 having optical transparency, and is used by winding it around the head of a user in a vertical direction (Fig. 4). Thus, each of the light-emitting parts 7 of the main bodies 2 is held facing the temporomandibular joint and its surrounding area of the user, and can irradiate light according to each of the modes.

The holder 41 is formed from a material such as polyester or polyurethane and has moderate elasticity and strength. Of course, the material for the holder 41 is not limited to these. In each end portion of the longitudinal direction of the holder 41, an engaging part 46 composed of a hook-and-loop fastener, hook, and the like is provided so that the light irradiation device 40 can be fitted and fixed to the face of the user.

The pocket 42 is configured by a cover 42a that has a substantially rectangular shape and is formed with a material having optical transparency transmitting the light of the light-emitting part 7, such as PET, PVC, PC, PMMA, TPU, etc. The pocket 42 is formed, for example, by attaching the cover 42a to the holder 41 through sewing or bonding, such that one edge is left open. The main body 2 can be inserted into and removed from the open end of the pocket 42 of the holder 41.

The holder 41 has terminal holes 43 inside the respective pockets 42, from each of which the connecting terminal part 11 of the main body 2 is exposed. When the main body 2 is housed in the pocket 42, the connecting terminal part 11 provided on the bottom surface 5b of the substrate 5 is exposed from the terminal hole 43, and the main body 2 is made to be connectable with the electric cable 15.

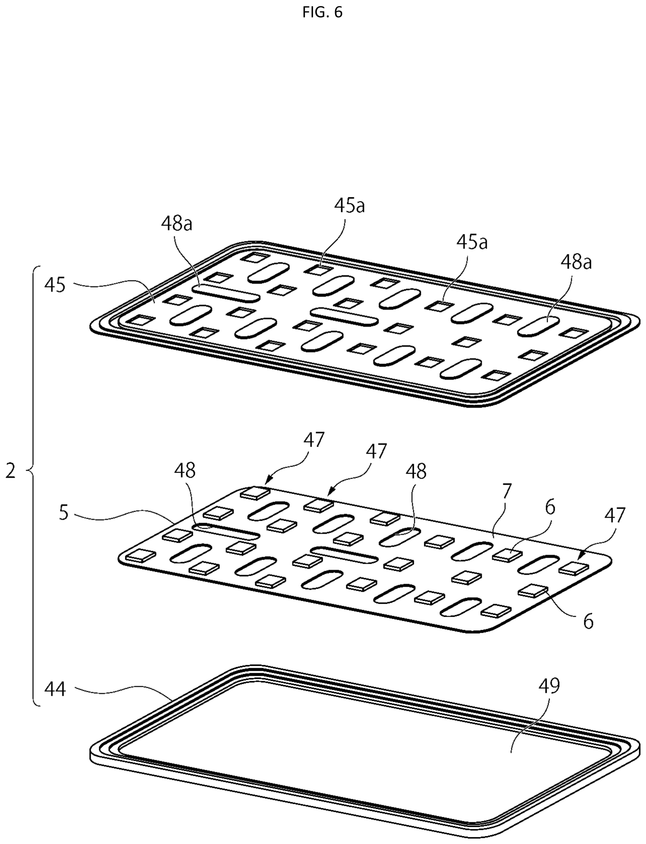

As shown in Fig. 6, the main body 2 used in the light irradiation device 40 includes the substrate 5 provided with the light-emitting part 7, and a tray 44 and a protective cover 45 that hold the substrate 5 from both sides. Since the substrate 5 is interposed between the tray 44 and the protective cover 45, it is easy to handle, and the light-emitting part 7 can be protected.

The substrate 5 has an approximately trapezoidal shape with rounded corners, and the top surface 5a has unit rows 47 arranged in six columns at predetermined intervals. Each unit row is configured from four or three units formed by combinations of the first light emitters 6a and the second light emitters 6b. The arrangement pattern of the light emitters 6 or units in the light irradiation device 40 is not limited to this. Additionally, oval opening portions 48 are provided between respective spaces of the unit rows 47.

The tray 44 has an approximately trapezoidal shape with rounded corners, the same as the substrate 5, and is provided with a housing concave portion 49 in which the substrate 5 is housed. The tray 44 can be formed by a resin material having flexibility, such as polyimide resin, fluororesin, silicone resin, PET resin, or combinations of these resins containing a white pigment (white resin, white resist, etc.), etc. Incidentally, the material for the tray 44 is not limited to these materials.

The protective cover 45 covers the top surface 5a of the substrate 5 housed in the tray 44, and is provided with oval convex portions 48a which are respectively inserted into the opening portions 48 provided on the substrate 5. The substrate 5 can be positioned by respectively inserting the convex portions 48a of the protective cover 45 into the opening portions 48. The protective cover 45 is provided with concave portions 45a for preventing interference with the light emitters 6 in accordance with the formation position of the light emitters 6 of the substrate 5. The protective cover 45 is formed from a resin material such as PET, PVC, PC, PMMA, or TPU, which have optical transparency to transmit light from the light-emitting part 7 and flexibility. Incidentally, the material for the protective cover 45 is not limited to these materials.

In the light irradiation device 40, since the main body 2 housed in the holder 41 and the holder 41 has flexibility, as shown in Fig. 4, the light-emitting part 7 of the main body 2 can adhere to the temporomandibular joint and its surrounding area of the user by winding and fitting the holder 41 longitudinally around the user's head. Therefore, the light irradiation device 40 can efficiently irradiate the affected part with light corresponding to each mode without compromising wearing comfort. Incidentally, the light irradiation device 40 may also be used on areas other than the head.

Examples of the settings of the wavelength, radiation illumination, irradiation time, and absorbed energy of the light emitters 6 in each mode of light irradiation are described below. The surface of the substrate 5 is provided with twenty-one pieces of LED units as the light emitters 6. Each unit is configured from the first light emitter 6a emitting red light (emission wavelength: 640 to 770 nm) and the second light emitter 6b emitting infrared light (emission wavelength: approximately 770 nm to 1,000 nm). The light irradiation device 40 has the substrate 5 mounted on each of the holders 41, with a total of forty-two LED units installed. Incidentally, in either mode, the operation stopping temperature of the light emitters 6 monitored by the temperature detection part 17 is set at, for example, 43°C.

In the first mode, only the red light is emitted by the first light emitters 6a. The operating current of each of the first light emitters 6a may be set in a range of 9 mA ± 10% (8.1 to 9.9 mA), the radiant flux of the per unit area may be set in a range of 14.0 mW/cm² ± 10% (12.6 to 15.4 mW/cm²), and the operating time may be set in a range of 360 sec ± 10% (324 to 396 sec). When the operating current is 9 mA, the radiant flux of the per unit area is 14.0 mW/cm², and the operating time is 360 sec, the total radiation illuminance of the red light irradiated from each of the main bodies 2 is 294.0 mW/cm², which is converted to 588.0 mW/cm² for the whole light irradiation device 40, and the absorbed energy (J) of the irradiation light from each of the main bodies 2 is 105.8 J, which is converted to 211.6 J for the whole light irradiation device 40.

In the second mode, only the infrared light is emitted by the second light emitters 6b. The operating current of each of the second light emitters 6b may be set in a range of 33 mA ± 10% (29.7 to 36.3 mA), the radiant flux of the per unit area may be set in a range of 65.4 mW/cm² ± 10% (58.9 to 71.9 mW/cm²), and the operating time may be set in a range of 360 sec ± 10% (324 to 396 sec). When the operating current is 33 mA, the radiant flux of the per unit area is 65.4 mW/cm², and the operating time is 360 sec, the total radiation illuminance of the infrared light irradiated from each of the main bodies 2 is 1372.9 mW/cm², which is converted to 2745.8 mW/cm² for the whole light irradiation device 40, and the absorbed energy (J) of the irradiation light from each of the main bodies 2 is 494.2 J, which is converted to 988.4 J for the whole light irradiation device 40.

In the third mode, the red light is emitted by the first light emitters 6a, and the infrared light is emitted by the second light emitters 6b. The operating current of each of the first light emitters 6a may be 7 mA ± 10% (6.3 to 7.7mA), the operating current of each of the second light emitters 6b may be 33 mA ± 10% (29.7 to 36.3mA), the radiant flux of the per unit area may be set in a range of 79.4 mW/cm² ± 10% (71.5 to 87.3 mW/cm²), and the operating time may be set in a range of 360 sec ± 10% (324 to 396 sec). When the operating current of the first light emitters 6a is 7 mA, the operating current of the second light emitters 6b is 33 mA, the radiant flux of the per unit area is 79.4 mW/cm², and the operating time is 360 sec, the total radiation illuminance of the infrared light irradiated from each of the main bodies 2 is 1666.9 mW/cm², which is converted to 3333.8 mW/cm² for the whole light irradiation device 40, and the absorbed energy (J) of the irradiation light from each of the main bodies 2 is 600.0 J, which is converted to 1200.0 J for the whole light irradiation device 40.

Incidentally, the radiation illuminance can be obtained by irradiating from the light emitters 6 and measuring the radiant flux of light incident per unit area with a radiation intensity meter. The total radiation illuminance from each of the main bodies 2 can be calculated by multiplying the radiation illuminance of each of the light emitters 6 and the number of light emitters 6. The absorbed energy (W·sec) of the light irradiated from each of the main bodies 2 can be calculated by multiplying the total radiation illuminance (W) and the irradiation time (sec).

The values of emission wavelength, radiation illuminance, operating time, and absorbed energy of the light emitters 6 for each mode described above are examples, and the setting values for each mode are not limited to those described above. For the light irradiation device 40, the emission color of an indicator in the information part 25 of the controller 3 may be changed for each of the first to third modes, so that it can be identified in which mode it is driven.

### [Second embodiment]

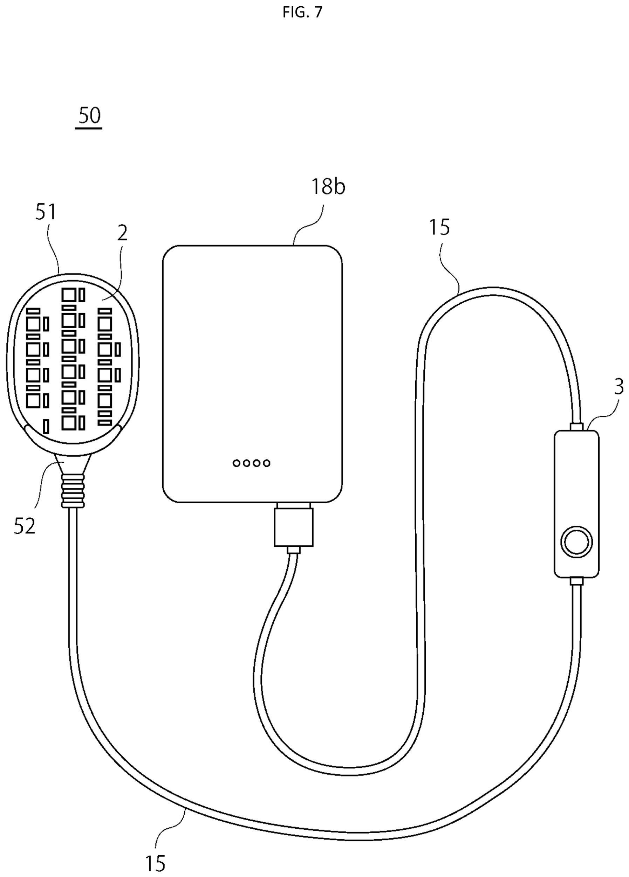

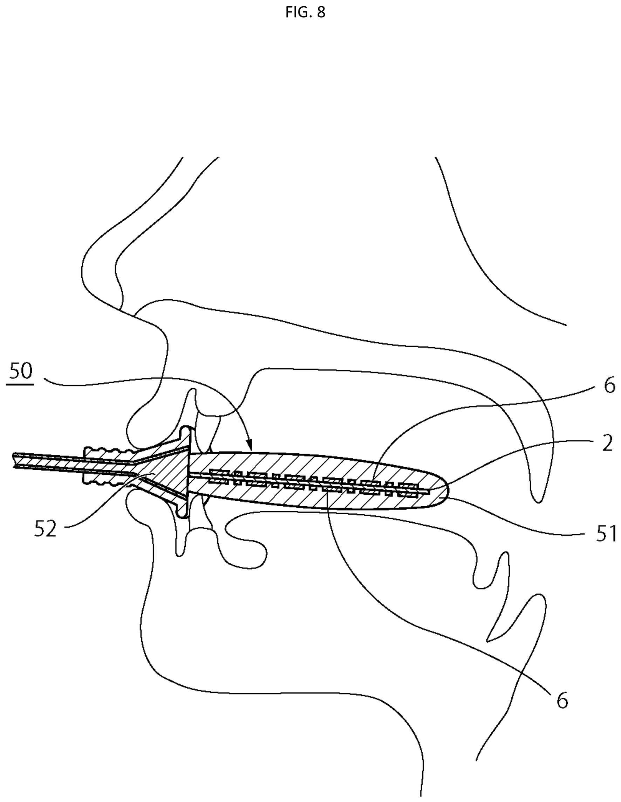

The second embodiment of the light irradiation device 1 is described below. The light irradiation device 50 of the second embodiment is used for mucous membrane treatment in the oral cavity. Fig. 7 is a top plan view showing the light irradiation device 50, and Fig. 8 is a drawing showing a using state of the light irradiation device 50. The light irradiation device 50 has an exterior material 51 incorporating the main body 2 and held in the oral cavity.

The exterior material 51 has an oval plate shape in a plan view, for example, and has a size that can be held between the upper side of the tongue and the palate in the oral cavity or between the outer side of the dentition and each cheek. The exterior material 51 has optical transparency in the part facing the light-emitting part 7 of the main body 2 at least, and is capable of radiating light from the light-emitting part 7. A lead-out part 52 is formed on one side edge portion along the longitudinal axis of the exterior material 51, from which the electric cable 15 is led out. In the main body 2, the connecting terminal part 11 formed on the substrate 5 is connected to the electric cable 15, and the electric cable 15 is led out from the lead-out part 52 and connected to the controller 3.

The material of the exterior material 51 is the same as that of a mouthpiece conventionally used in the field of sports and medicine, and is formed of a transparent or translucent resin material capable of irradiating light from the light source 7 and having flexibility and shape retention properties. Specifically, the material for the exterior material 51 can be a resin that conforms to the shape of the oral cavity and has appropriate hardness and softness, such as olefin-based resins, polyester-based resins, urethane-based resins like polyurethane, polyimide-based resins, silicone-based resins, styrene-based resins, acrylic-based resins, polyamide-based resins, and carbonate-based resins. However, it is not limited to these materials.

As the above-described olefin-based resins, the following are preferably exemplified: polyethylene (PE), polyethylene-based resins, polypropylene (PP), polypropylene-based resins, ethylene-vinyl acetate copolymer (EVA), etc., and the following are more preferably exemplified: polyethylene (PE), polyethylene-based resins, polypropylene (PP), polypropylene-based resins, etc.

Polyester-based resins are polycondensates of polycarboxylic acids (such as dicarboxylic acids) and polyalcohol (such as diols). As the above-described polyester-based resins, polyethylene terephthalate (PET) is exemplified.

Urethane-based resins are polycondensates formed from a compound containing isocyanate groups and a compound containing hydroxyl groups. As the above-described urethane-based resins, thermoplastic polyurethane (TPU) is exemplified.

Polyamide-based resins are (co)polymers formed by the bonding of numerous monomers via amide bonds. As the above-described polyamide-based resins, there are examples, such as nylon, para-type amides, and meta-type amides.

Acrylic rubber-based resins are (co)polymers containing acrylic-based rubbers as main components. As the above-described acrylic-based resins, there are examples, such as a block copolymer of methyl methacrylate and butyl acrylate.

Incidentally, the exterior material 51 may be configured from multiple parts, and each of the parts may be formed from different materials to vary their hardness.

The exterior material 51 can be obtained by molding the above-described material integrally with the main body 2. An injection condition for the material is set according to the size and shape of the exterior material 51. When the exterior material 51 is configured by a single material, the entire exterior material 51 is molded in a single mold. The size of the exterior material 51 may be suitable for conforming to the inside of the oral cavity of an average person. The light irradiation device 50 is manufactured to have a size corresponding to the oral cavity of an average adult when the wearer is an adult, and is manufactured to have a size corresponding to the oral cavity of an average child when the wearer is a child.

When the exterior material 51 is configured from multiple parts, for example, it can be formed by molding each part, and then interposing the main body 2 between the parts and bonding or welding them together.

The light irradiation device 50 is held between the upper side of the tongue and the palate, or between the outer side of the dentition and each cheek in the oral cavity so as to face the light-emitting part 7 to the palate and the inside of each cheek. Then, the light irradiation device 50 can irradiate the light to the affected part on the palate or the inside of each cheek according to each of the modes.

Incidentally, the light irradiation device 50 may also be provided with the light-emitting part 7 on the bottom surface 5b side of the substrate 5 of the main body 2, and form the exterior material 51 such that the portion facing the light-emitting part 7 on the bottom surface 5b side is optically transparent. Thus, the light irradiation device 50 can irradiate the light to both the affected parts on the upper portion of the tongue and the palate, or the respective affected parts on the inside of each cheek and gums.

The setting values for the wavelength, radiation illuminance, irradiation time, and absorbed energy of the light emitters 6 in each of the modes of the light irradiation of the light irradiation device 50 can be set in the same way as those described for the light irradiation device 40, though this is by no means exclusive.

### REFERENCE SIGNS LIST

- 1: light irradiation device
- 2: main body
- 3: controller
- 5: substrate
- 6: light emitters
- 6a: first light emitters
- 6b: secondlight emitters
- 7: light-emitting part
- 8: control part
- 11: connecting terminal part
- 15: electric cable
- 17: temperature detection part
- 18: power supply unit
- 21: operating part
- 22: setting part
- 24: display part
- 25: information part
- 26: communication part
- 28: microprocessor
- 29: memory
- 31: server
- 32: power switch
- 33: control switch
- 34: storage
- 35: detection circuit
- 40: light irradiation device
- 41: holder
- 42: pocket
- 43: terminal hole
- 44: tray
- 45: protective cover
- 45a: concave portion
- 47: unit row
- 48: opening portion
- 48a: convex portion
- 49: housing concave portion
- 50: light irradiation device
- 51: exterior material
- 52: lead-outpart

## Claims

1. A light irradiation device irradiating light to an affected part, comprising:
a main body comprising a substrate, and a light-emitting part that is disposed on at least one surface of the substrate and includes a plurality of light emitters with different emission wavelengths, and
a controller equipped with a control part that controls light emission from the light-emitting part,
wherein the light-emitting part at least includes the first light emitters emitting light with a wavelength in the first band and the second light emitters emitting light with a wavelength in the second band, different from the first band, and
wherein the control part executes an operation by switching at least between the first mode in which the first light emitters are made to emit, the second mode in which the second light emitters are made to emit, and the third mode in which the first light emitters and the second light emitters are made to emit.

2. The light irradiation device according to claim 1, wherein the first band is within a range of 640 to 770 nm, and the second band is within a range of 770 to 1,000 nm.

3. The light irradiation device according to claim 1 or 2, wherein an output of the light of the wavelength in the first band is different between the first mode and the third mode.

4. The light irradiation device according to claim 1 or 2, wherein the light-emitting part includes the third light emitters emitting light with a wavelength in the third band different from the first and second bands, and wherein the third band is within a range of 430 to 490 nm.

5. The light irradiation device according to claim 1 or 2, wherein the substrate has flexibility and is configured to be capable of adhering to the affected part.

6. The light irradiation device according to claim 5, wherein a holder having a pocket in which the main body is housed and fit to the affected part is further provided, and wherein the pocket is configured with a material that transmits the light of the light-emitting part to be capable of irradiating the light to the affected part of a wearer to which the holder is adhered.

7. The light irradiation device according to claim 6, wherein the holder is fit to the face, and the light is irradiated to an area including the temporomandibular joint and its surrounding area from a surface of skin.

8. The light irradiation device according to claim 1 or 2, wherein the main body is covered with an exterior material formed with a resin material, a surface of the exterior material facing at least the light-emitting part is optically transparent, and the light is possible to be irradiated through the exterior material.

9. The light irradiation device according to claim 8, wherein the light-emitting part is disposed on the other surface opposite to said one surface of the substrate, too.

10. The light irradiation device according to claim 8, wherein the device is held between the upper side of a tongue and a palate in an oral cavity or between the outside of dentition and each cheek to irradiate the light in the oral cavity.
